# EUROPEAN PATENT APPLICATION

(11) **EP 1 524 269 A1**
(43) Date of publication of application: **20.04.2005**
(21) Application number: 03090335.5
(22) Date of filing: 07.10.2003
(51) Int. Cl.: C07D 311/32, A61K 31/35, A61P 5/00

(54) **Use of 8-Prenylnaringenin for hormone replacement therapy**

(71) Applicant: Schering Aktiengesellschaft, 13353 Berlin (DE)
(72) Inventor: Hümpel, Michael, 14163 Berlin (DE); Schleuning, Wolf-Dieter, 13467 Berlin (DE); Schaefer, Olaf, 10779 Berlin (DE); Isaksson, Päivi, 25700 Kimito (FI); Bohlmann, Rolf, 14055 Berlin (DE)

(57) **Abstract**

The invention provides a production method for the phytoestrogen 8-Prenylnaringenin, the preparation produced by this method and the use of 8-Prenylnaringenin for the production of a medicament for the prevention and treatment of hormone-dependent osteoporosis and of peri- and postmenopausal symptoms in women.

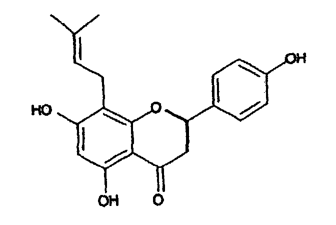

## Description

This invention is directed to a production method of 8-Prenylnaringenin, the preparation produced by this method and its use for the production of a medicament.

Hormone replacement therapy is used for the treatment of menopausal symptoms (short term) and for the prevention or treatment of hormone dependent osteoporosis (short and long term). The hormones may be estradiol, estrone, estriol, ethinyl estradiol or conjugated estrogens. All these estrogens are effective in the treatment of menopausal symptoms and the prevention of osteoporosis but also exhibit a non-dissociated proliferative effect on uterine tissue. This proliferation is associated with increased risk for endometriosis and endometrial cancer and lead to vaginal bleedings as a progestin is necessary from time to time to counteract the estrogenic effects on the uterus. Therefore, there is a clear medical need for estrogens which can treat menopausal symptoms and prevent or treat bone loss but only exert minimal or very low proliferative effects in the uterus. Continous treatment with such estrogens would not need a counteracting progestin at the level of uterine tissue. Some synthetic antiestrogens, e.g. Tamoxifen and Raloxifen, have been synthesized which show a certain tissue selectivity (Evans ans Turner, Bone 17, 181 S-190S, 19995). Also plant secondary metabolites occurring compounds were identified which show some estrogenic activity e.g. isoflavones, lignans and others. These so-called phyto-estrogens are, however, very weak estrogens and rather high daily doses seem to be required for clinical effects. As it was demonstrated that there is a substantial reduction in breast-cancer risk among women with a high intake of phytoestrogens (Ingram et al 1997, The Lancet 350, 990-994), it would be desirable to find a phyto-estrogen which shows a tissue selectivity in estrogenic effects and thus is able to treat menopausal symptoms and prevent hormone dependent osteoporosis without activating uterine tissue to a degree that would need a counteracting progestin in continous treatment regimens.

The phyto-estrogen 8-Prenylnaringenin, derived from the cones of hop (Humulus lupulus) was reported to have a pronounced estrogenic activity which is at least one order of magnitude higher than that of the isoflavone genistein. As 8-Prenylnaringenin is the most potent phyto-estrogen known, this compound clearly is a good candidate for the preparation. It was, however, reported that 8-Prenylnaringenin did not show the desired tissue selectivity but has a significant effect on uterus growth at the bone loss protective dose in an animal study (Miyamoto et al 1998, Planta Medica 64, 516-519). According to these results this compound would not be chosen as the desired drug candidate.

Surprisingly, an animal study performed with a synthetic relatively pure 8-Prenylnaringenin preparation revealed that 8-Prenylnaringenin exerts selective effects on bone and uterus. While it prevents bone loss in a dose dependent manner, it has only a minimal and dose independent effect on uterus growth and endometrial stimulation. The effect of said preparation on uterus proliferation is less than 1/5 and probably 1/10 of that of estradiol wherein doses of 8-Prenylnaringenin and estradiol are compared which are equi-effective on protection of bone loss. The calculation of the ratio of the effect on uterus growth and protection of bone loss is illustrated by the following example: the effect on uterus weight of 0.4 µg/kg/d estradiol is similar to that of 18 mg/kg/d 8-prenylnaringenin, thus the dose ratio is 18 000:0.4 = 1:45 000. The protection of bone loss of 4 µg/kg/d estradiol is similar to that of 18 mg/kg 8-Prenylnaringenin, thus the dose ratio is 18 000:4 = 1:4500. These data were measured according to example 1 and 2 and may vary within normal statistical limits. Extending this calculation to the human situation, the treatment of women with a bone protective dose of 8-Prenylnaringenin would result in an estrogenic effect on the uterus that would be equivalent to an oral estradiol dose of 0.1-0.2 mg/d and thereby fall below the no effect level. This means that peri- and postmenopausal symptoms might be treated without recurrence of vaginal bleedings.

8-Prenylnaringenin (5,7-Dihydroxy-2-(4-hydroxyphenyl)-8-(3-methylbut-2-enyl)-chroman-4-on) has the following structure:

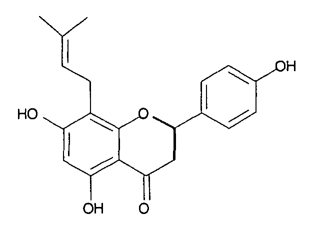

The 4-hydroxyphenyl group may either be in the 2S(-) or the 2R (+) position. Either one of the enantiomers or the racemate may be used. The effect on uterus proliferation and the protection of bone loss may be measured in rats as described in example 2 (uterus weight, histology of endometrium and bone mineral density measurement).

The 8-Prenylnaringenin preparation is synthesized by a method which is based on the method described by Gester et al (2001, Tetrahedron 57, 10115-1018) but includes several improvements. One embodiment of this invention is therefore a method to synthesize 8-Prenylnaringenin comprising the steps:
a. diacetylation of naringenin, crystallization of product
b. prenylation using tributylphosphine and diisopropylazodicarboxylate, crystallization of product
c. rearrangement of prenyl side chain using Europium(III)-fod as catalyst, product as residue after destillation of solvent and
d. solvolysis using K₂CO₃ in methanol/H₂O, product by extraction and various washing procedures
whereby said steps do not include a chromatography.

Because no chromatographic steps are needed and the amount of catalyst is drastically reduced, this method is much faster and cheaper than the previously described method, which - by ecomonical reasons - is not a suitable method for up-scaled production.

The invention also relates to the 8-Prenylnaringenin preparation produced by the method of this invention. This preparation is at least 95% pure. The purity may be determined by HPLC.

A further embodiment of this invention is the use of 8-Prenylnaringenin for the production of a medicament for the prevention and treatment of osteoporosis and of peri- and postmenopausal symptoms in women wherein the 8-Prenylnaringenin preparation used for the production is at least 95% pure.

This invention also relates to the use of the 8-Prenylnaringenin preparation synthesized by the method of this invention for the production of a medicament for the prevention and treatment of osteoporosis and of peri- and postmenopausal symptoms in women.

Peri- and postmenopausal symptoms are for example hot flushes, mood changes, night sweats and vaginal dryness.

Administration of the medicament of the invention can be carried out via any of the accepted modes of administration or agents for serving similar utilities.Thus, administration can be, for example, orally, nasally, parenterally, topically, transdermally, or rectally, sublingually, intramuscular, subcutaneously, or intravenously in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and 8-Prenylnaringenin as the/an active agent, and, in addition, may include other medicinal agents, pharmaceutical agents, carriers, adjuvants, etc.

Generally, depending on the intended mode of administration, the pharmaceutically acceptable compositions will contain about 1% to about 99% by weight of 8-Prenylnaringenin and 99% to 1% by weight of one or more suitable pharmaceutical excipient(s). Preferably, the composition will be about 5% to 75% by weight of 8-Prenylnaringenin, with the rest being suitable pharmaceutical excipients.

The preferred route of administration is oral, using a convenient daily dosage regimen which can be adjusted according to the degree of severity of the disease-state to be treated. For such oral administration, a pharmaceutically acceptable composition containing 8-Prenylnaringenin is formed by the incorporation of any of the normally employed excipients. Such excipients include non-toxic and chemically compatible fillers, binders, disintegrants, buffers, preservatives, anti-oxidants, lubricants, flavorings, thickeners, coloring agents, emulsifiers, and the like, for example, pharmaceutical grades of mannitol, lactose, starch, pregelatinized starch, magnesium stearate, sodium saccharine, talcum, cellulose ether derivatives, glucose, gelatin, sucrose, citrate, cyclodextrin, propyl gallate, and the like. Such compositions take the form of solutions, suspensions, tablets, pills, capsules, powders, sustained release formulations and the like.

Preferably such compositions will take the form of capsule, caplet or tablet and therefore will also contain a diluent such as lactose, sucrose, dicalcium phosphate, and the like; a disintegrant such as croscarmellose sodium or derivatives thereof; a lubricant such as magnesium stearate and the like; and a binder such as a starch, gum acacia, polyvinylpyrrolidone, gelatin, cellulose ether derivatives, and the like.

8-Prenylnaringenin may also be formulated into a suppository using, for example, about 0.5% to about 50% active ingredient disposed in a carrier that slowly dissolves within the body, e.g., polyoxyethylene glycols and polyethylene glycols (PEG), e.g., PEG 1000 (96%) and PEG 4000 (4%), and propylene glycol.

Liquid pharmaceutically administrable compositions can, for example, be prepared by dissolving, dispersing, etc., 8-Prenylnaringenin (about 0.5% to about 20%), and optional pharmaceutical adjuvants in a carrier, such as, for example, water, saline, aqueous dextrose, aqueous cyclodextrin, glycerol, ethanol and the like, to thereby form a solution or suspension.

If desired, a pharmaceutical composition of the invention may also contain minor amounts of auxiliary substances such as wetting or emulsifying agents, pH buffering agents, antioxidants, and the like, such as, for example, citric acid, sorbitan monolaurate, triethanolamine oleate, butylated hydroxytoluene, etc.

Actual methods of preparing such dosage forms are known, or will be apparent, to those skilled in this art; for example, see *Remington's Pharmaceutical Sciences,* 18th Ed., (Mack Publishing Company, Easton, Pennsylvania, 1990). The composition to be administered will, in any event, contain a therapeutically effective amount 8-Prenylnaringenin for prevention and treatment of bone loss or of peri-and postmenopausal symptoms in women.

8-Prenylnaringenin is administered in a therapeutically effective amount which will vary depending upon a variety of factors including the age, body weight, general health, diet, mode and time of administration, rate of excretion, drug combination, the severity of the particular disease-states, and the host undergoing therapy. Generally, a therapeutically effective daily dose is from about 0.5 to about 25.0 mg/kg of body weight per day 8-Prenylnaringenin preferably, from about 1 mg to about 10 mg/kg of body weight per day; and most preferably, from about 2 mg to about 5 mg/kg of body weight per day.

### Example 1: Method to synthesize 8-Prenylnaringenin

The total method of synthesis is in four steps.

### Step 1: Acetylation of Naringenin

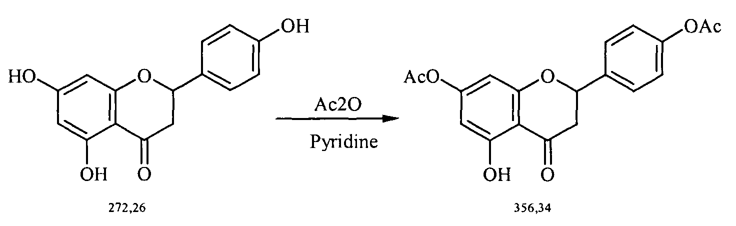

100 g of naringenin (available as bulk ware from various suppliers) are mixed with 200 g pyridine (analytical grade) at room temperature and 75 g acetic anhydride (analytical grade) is added dropwise under cooling and stirring within 90 minutes. At the end of process the diacetylated product starts to precipitate and stirring is continued for another 15 minutes. Then the mixture is poured to 1 L icecold water and the precipitate is filtered. The wet product is then washed with 320 ml of 0.1 N HCl and icecold water. The water-wet product is then boiled with 350 ml methanol and filtered twice and finally washed with 50 ml of methanol. The product is dried in vacuo at 40 °C over night. The product is pure by > 82.5 %. The yield is about 107 g of diacetylated product (I).

### Step 2: Prenylation of diacetylated product (I)

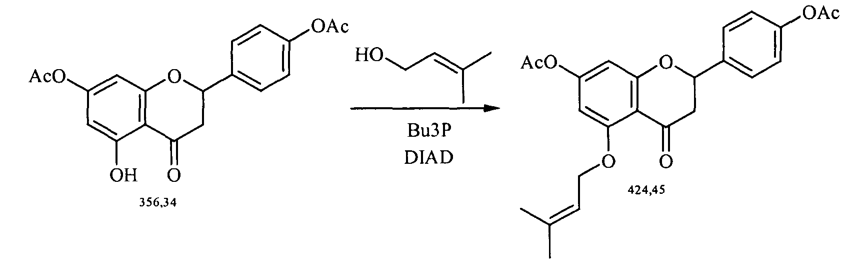

106.9 g of I and 36.2 g 3-methyl-2-buten-1-ol (98 %) are mixed with 2670 ml of THF (analytical grade, dried over molecular sieve) and cooled down to 0 °C under nitrogen (ice bath). 74 g of tributylphosphin (98 %) are added under nitrogen. A solution of 95.8 g of diisopropylazodicarboxylate (97 %) in 425 ml of dry THF is then added dropwise at 0 °C within 120 minutes. Following the complete reaction THF is removed by distillation and the oily residue taken up in 500 ml toluene. This solution is washed twice with each 150 ml of water and five times with each 150 ml of 0.8 M Na₂CO_{3.} Following phase separation toluene is removed by distillation and the oily residue is dissolved in 300 ml tert. Butylmethylether or another suitable solvent. At -10°C and the addition of primer crystals the prenylated product (II) starts to precipitate after 1-2 hours. To complete crystallisation stirring under cooling is continued for another 16 hours. The product is then filtered, washed with 80 ml of ether or suitable solvent and dried in vacuo at 40 °C over night. The yield is 39.1 g corresponding to 30.7 %. The product is > 78 % pure.

### Step 3: Claisen rearrangement of II

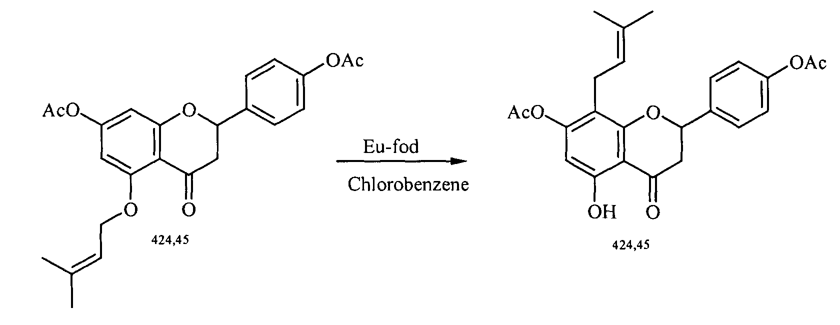

36 g of product II, 4.4 g of Eu(III)-fod (99 %) and 180 ml of chlorobenzene (synthetical grade) are mixed and heated to 75-80 °C for about 5 hours. Reaction is followed by HPLC and after completion solvent is removed by distillation. The obtained product III is directly used for step 4. Yield is 41 g of wet product.

### Step 4: Deacetylation

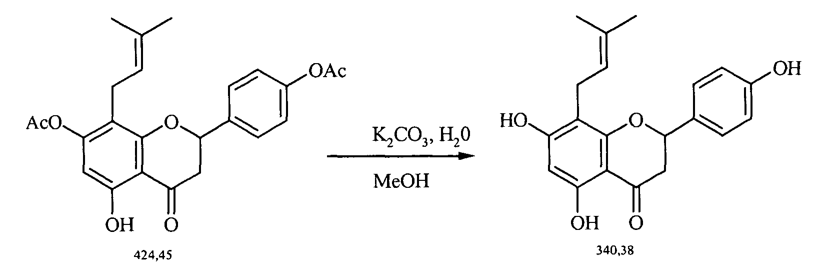

41 g of wet product III are mixed with 410 ml of methanol and 20 ml of deionized water. 2.25 g of potassium carbonate are added and the reaction mixture warmed to 40 °C for about 18 hours. After reaction is completed 400 ml of water are added and the mixture adjusted to pH 7 with 1 M HCl. By this, the final compound IV precipitates. Methanol is then removed by distillation at 40 °C and the aqueous phase containing the precipitate is extracted with 300 ml tert. Butylmethylether or another suitable solvent. Extraction is repeated twice with each 100 ml. Combined organic phases are washed with 200 ml water and then evaporated to dryness. The raw product is then stirred with 250 ml dichlormethane, filtered and again washed with 250 ml dichlormethane and dried at 40 °C. The raw product is then dissolved in 250 ml tert. Butylmethylether or another suitable solvent, separated from insolubles by filtration and evaporated. Residues are suspended in a small amount of dichlormethane, filtrated and the filtrate dried in vacuo at 40 °C over night. Yield is 15.9 g slightly yellowish powder with a purity of > 95 %.

### Example 2:

### Effect of estradiol on bone mineral density and uterine weight in ovariectomized rats

### Study objectives

The aim of the study was to find the optimal protective estradiol dose against ovariectomy induced bone loss and also to compare three different vehicle solutions (benzyl-benzoate/ricinus oil vs. Ethanol/arachis oil and benzylbenzoate/rcinus oil vs. Benzyl alcohol/sesame oil).

### Description of study

Fifty-six female Sprague-Dawley rats, 12-14 weeks old, were used altogether. Forty-nine animals were randomized into seven groups. Group 8 (seven animals) administered with the third vehicle, benzyl alcohol/sesame oil, was added to the study thirteen days later. Seven groups were ovariectomized (OVX) and one group was sham-operated (SHAM). Animals were treated subcutaneously once a day, five days a week (Monday-Friday) as follows. Groups 1 (the SHAM group) and 2 (the OVX group) were given the vehicle benzylbenzoate/ricinus oil (0.5ml/kg, B/R). Groups 3-6 were given 17β-estradiol (Sigma) 0.2, 0.4, 1.2 and 4.0µg/0.5ml/kg in vehicle B/R, respectively. Group 7 given 17β-estradiol (Sigma) 1.2µg/0.5ml/kg in vehicle ethanol/arachis oil (E/A) and group 8 was administered 17β-estradiol hemihydrate (Schering AG, Berlin) 1.2µg/0.5mg/kg in vehicle benzyl alcohol/sesame oil (BAS/S). Treatments started on the day following surgical operation and continued for 4 weeks. At the end of the study, *ex vivo* BMD of proximal tibia was measured and serum estradiol was determined. In addition, uterus was weighed.

### Conclusions

The optimal protective subcutaneous dose of 17β-estradiol seemed to be 4.0µg/kg/d when administered five times a week for four weeks. This dose prevented the OVX-induced decrease in total and trabecular bone mineral density of proximal tibia and it also prevented the decrease in relative uterine weight and maintained it on the level of the SHAM vehicle group.

**Table 1**

| **Initial and final body weights, and change in body weight after 4 weeks treatment**. | | | | | |
|---|---|---|---|---|---|
| Group No | Operation/ Treatment | Dose µg/kg/d | Initial body weight g | Final body weight g | The change in body weight g |
| 1 | SHAM/Veh B/R | | 257 (7) | 268 (5) | 11 ( 3) |
| 2 | OVX/Veh B/R | | 252 (6) | 316 (8) | 65 ( 4) |
| 3 | OVX/Est in B/R | 0.2 | 252 (7) | 304 (6) | 51 (3) |
| 4 | OVX/Est in B/R | 0.4 | 259 (6) | 312 (6) | 53 ( 5) |
| 5 | OVX/Est in B/R | 1.2 | 255 (5) | 303 (6) | 48 ( 4) |
| 6 | OVX/Est in B/R | 4.0 | 253 (6) | 284 (7) | 31 ( 3) |
| 7 | OVX/Est in E/A | 1.2 | 254 (7) | 287 (8) | 33 ( 3) |
| 8 | OVX/Est in BA/S | 1.2 | 260(11) | 288(11) | 28 ( 1) |

**Table 2**

| **Total and trabecular bone mineral density of proximal tibia and relative uterine weight after 4 weeks treatment.** | | | | | |
|---|---|---|---|---|---|
| Group No | Operation/ Treatment | Dose µg/kg/d | Total bone density mg/cm³ | Trabecular density mg/cm³ | Relative uterine weight % of body weight |
| 1 | SHAM/Veh B/R | | 622 (20) | 431 (25) | 0.169 (0.020) |
| 2 | OVX/Veh B/R | | 485 (18) | 243 (27) | 0.036 ( 0.003) |
| 3 | OVX/Est in B/R | 0.2 | 511 (17) | 271 (17) | 0.045 ( 0.003) |
| 4 | OVX/Est in B/R | 0.4 | 506 (8) | 260 (13) | 0.054 ( 0.003) |
| 5 | OVX/Est in B/R | 1.2 | 577 (24) | 376 (31 ) | 0.110 (0.008) |
| 6 | OVX/Est in B/R | 4.0 | 637 (13) | 435 (21) | 0.159 (0.006) |
| 7 | OVX/Est in E/A | 1.2 | 577 (22) | 366 (24) | 0.114 (0.005) |
| 8 | OVX/Est in BA/S | 1.2 | 596 (18) | 386 (21) | 0.131 (0.008) |

### Example 3:

### Effect of Phytoestrogen 2S(-) 8-PN on Bone Mineral Density and Uterus Weight in Ovariectomized Rats

The aim of the study was to examine bone and uterine effects of a 4-week treatment with 2S(-) 8-Prenylnaringenin (8-PN) in 3-month-old ovariectomized rats. 2S(-) 8-PN at the dose levels of 0.67 mg/kg, 1,77 mg/kg and 18 mg/kg was administered subcutaneously (s.c.), once a day, seven times a week. At the end of the study, uterine weight and *ex vivo* bone mineral density (BMD) of tibia were measured.

### Description of study

A total of 30 female 13-14-week-old Sprague-Dawley rats were used in the study. The animals were randomized into 5 groups, with 6 rats /group. Group 1 was sham-operated (SHAM) and groups 2-5 were ovariectomized (OVX).

The mean body weight of the animals was 271 g (SD 9, SE 2) before operation. Treatments with the vehicle or test substance started on the day following operation. The animals in the SHAM group received the vehicle, 30% hydroxypropyl-β-cyclodextrin s.c., and in the OVX groups either the vehicle or 2S(-) 8-PN at nominal doses of 0.67 mg/kg/d, 1.77 mg/kg/d or 18 mg/kg/d s.c. Treatments continued for 4 weeks; the dosing frequency was seven times a week. The animals were sacrificed one day after the last dosing. At the end of the study, left tibiae were excised for *ex vivo* BMD measurement (pQCT) at proximal tibia and uteri were excised for the determination of absolute and relative weights. In addition, samples for the determination of 2S(-)8-Prenylnaringenin concentration were taken from all dosing solutions on the day when they were prepared and on the last day of use. The data of body weights, relative uterine weights and BMD were analyzed with one-way analysis of variance.

### Bone densitometry ex vivo

Bone mineral density measurement was carried out by pQCT (XCT-960A, Stratec, Germany). The bone specimens were thawed before measurement. Six 1 mm-thick sections of the proximal tibia (2 mm below the epiphyseal growth plate) were scanned at 0.5 mm intervals using a voxel size of 0.148 mm. Total and trabecular BMD (mg/cm³) were measured. After bone densitometry, the specimens were wrapped back to saline saturated gauzes and stored at -20°C.

### Relative uterine weight was calculated as percentage of body weight

A post-study histological examination was performed. The formalin fixed uteri were embedded in paraffin, cut into 5 µm transverse sections and were stained with haematoxylin eosin. The sections were then evaluated quantitatively for luminal epithelium cell height.

### Summary of results

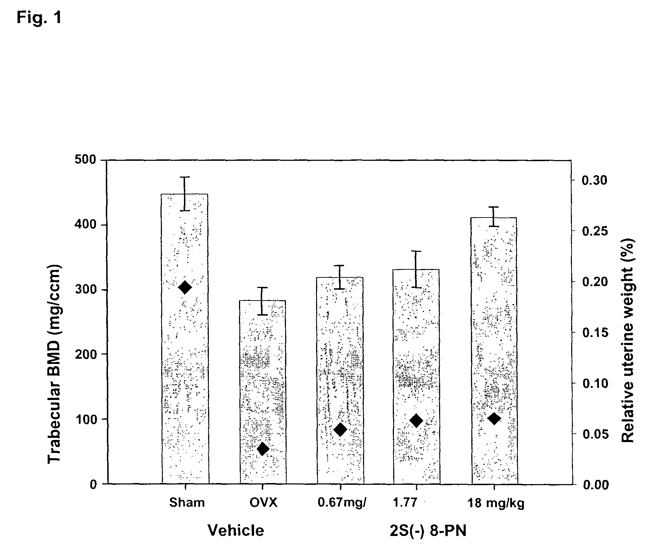

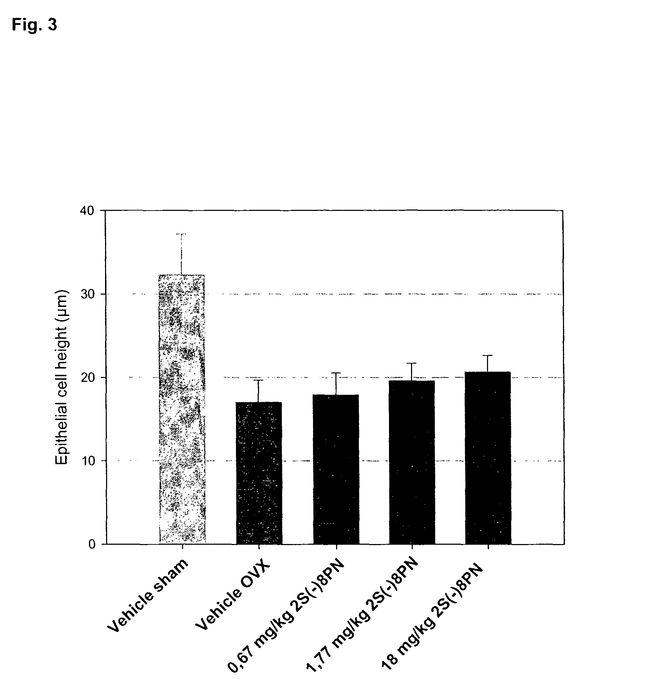

The results are shown in Fig.1 and Fig.3 and in the following tables:

**Table 3**

| **Body weight** | | | | | |
|---|---|---|---|---|---|
| Group | Operation/ Treatment Dose mg/kg/d | n | Body weight at the beginning (g) (bw0) | Body weight at the end (g) (bw4b) | The change in body weight (g) (bw4b-bw0) |
| 1 | Sham vehicle | 6 | 271 (3) | 278 (5) | 7 ( 3) |
| 2 | OVX vehicle | 6 | 272 (5) | 329 (5) | 57 ( 4) |
| 3 | 2S(-) 8-PN 0.67 | 6 | 270(4) | 323(11) | 53(7) |
| 4 | 2S(-) 8-PN 1.77 | 6 | 270 (4) | 305 (7) | 35 ( 5) |
| 5 | 2S(-) 8-PN 18 | 6 | 271 (4) | 304 (5) | 33 ( 4) |

**Table 4**

| **Relative uterus weight (%)** | | | | | | |
|---|---|---|---|---|---|---|
| | UTERUS WEIGHT | | | | | |
| | N | MEAN | STD | STDERR | MIN | MAX |
| GROUP | | | | | | |
| Sham vehicle | 6 | 0.194 | 0.035 | 0.014 | 0.154 | 0.234 |
| OVX vehicle | 6 | 0.035 | 0.004 | 0.002 | 0.029 | 0.041 |
| 2S (-) 8-PN 0.67 | 6 | 0.054 | 0.011 | 0.005 | 0.042 | 0.073 |
| 2S (-) 8-PN 1.77 | | 6 0.063 | 0.008 | 0.003 | 0.056 | 0.077 |
| 2S (-) 8-PN 18 | 6 | 0.065 | 0.009 | 0.004 | 0.051 | 0.078 |

**Table 5**

| **Parameters of ex vivo bone densitometry at left proximal tibia** | | | | | | | |
|---|---|---|---|---|---|---|---|
| Total BMD (mg/cm³) | | | | | | | |
| | N | MEAN | SE | STD | MEDIAN | MIN | MAX |
| Group | | | | | | | |
| Sham vehicle | 6 | 653.7 | 11.5 | 28.2 | 640.2 | 631.0 | 696.0 |
| OVX vehicle | 6 | 532.9 | 14.6 | 35.7 | 528.3 | 491.1 | 577.4 |
| 2S (-) 8 PN 0.67 | 6 | 561.1 | 11.3 | 27.6 | 569.2 | 524.5 | 591.1 |
| 2S (-) 8 PN 1.77 | 6 | 581.5 | 19.9 | 48.6 | 568.1 | 543 | 677.2 |
| 2S (-) 8 PN 18 | 6 | 636.5 | 17.8 | 43.6 | 635.4 | 582.8 | 699.2 |

| Trabecular BMD (mg/cm³) | | | | | | | |
|---|---|---|---|---|---|---|---|
| | N | MEAN | SE | STD | MEDIAN | MIN | MAX |
| Group | | | | | | | |
| Sham vehicle | 6 | 447.4 | 25.2 | 61.7 | 439.6 | 387.1 | 536.4 |
| OVX vehicle | 6 | 282.2 | 21.8 | 53.3 | 273.1 | 214.5 | 353.1 |
| 2S (-) 8 PN 0.67 | 6 | 319.1 | 17.4 | 42.7 | 318.7 | 250.3 | 379.8 |
| 2S (-) 8 PN 1.77 | 6 | 330.9 | 27.8 | 68.1 | 329.4 | 223.9 | 436.1 |
| 2S (-) 8 PN 18 | 6 | 411.8 | 15.2 | 37.2 | 396.6 | 378.7 | 474.0 |

Within this dose range 2S(-) 8-PN prevented the OVX-induced decreases in both total and trabecular BMD at the proximal tibial metaphysis dose dependently. The highest dose of 18 mg/kg/d completely inhibited OVX-induced effects.

Within the dose range 2S(-) 8-PN increased the relative uterine weight less than twofold when compared to ovariectomy. Compared to the SHAM group the relative uterine weights were markedly reduced ( 0.054-0.065 % vs 0.194 %).

The treatment of ovariectomized rat with ZK 222660 resulted in a weak stimulation of luminal epithelial cell height at 1.77 mg/kg and 18 mg/kg.

Comparing the effects of 2S(-) 8-PN on inhibition of bone loss and the increase in uterine weight to data with 17β-estradiol, 2S(-) 8-PN has a much lower effect on the uterus than 17β-estradiol at the same bone effective dose.

### Example 4:

### Effects of 8-Prenylnaringenin (racemic), 2S(-) 8-Prenylnaringenin and Estradiol hemihydrate on bone mineral density and uterine weight in ovariectomized rats

The aim of the study was to directly compare the effects of Prenylnaringenin (racemate) and 2S(-) 8-Prenylnaringenin to the effects of estradiol hemihydrate on bone mineral density and uterine weight on 3-month-old ovariectomized rats using identical formulations. In addition, the effects of estradiolhemihydrate in two vehicles (oily, aqueous) were examined for influence of formulations.
The substances were administered subcutaneously(s.c.) seven days a week beginning the day after ovariectomy. At the end of the study, uterine weight and bone mineral density (BMD) of tibia were measured.

### Description of study

Forty-one female Sprague-Dawley rats, 12-14 weeks of age were randomized into six groups, 6-8 animals in each. Five groups were ovariectomized (OVX) and one group was sham-operated (SHAM). Treatments started on the day following operation and continued for 28 days. The animals were treated s.c. once a day seven times a week as follows. Group 1 (SHAM vehicle group) and group 2 (OVX vehicle group) were given the vehicle 30% hydroxypropyl-β-cyclodextrin (HP-β-CD). Groups 3 and 4 were administered 4 µg/kg/d of estradiol hemihydrate in vehicle benzyl benzoate/ricinus oil (B/R) and in vehicle HP-β-CD, respectively. Groups 5 and 6 were given 20 mg/kg/d of 2S(-)8-Prenylnaringenin and racemic 8-Prenylnaringenin, respectively, both in vehicle HP-β-CD. At the end of the study, left tibiae were excised for the *ex vivo* BMD measurement (pQCT) and uteri for the determination of absolute and relative weights. In addition, samples for thedetermination of test substance and control substance concentrations were taken from all dosing solutions on the first and last day of use. The data of body weights, relative uterine weights and BMD were analyzed with one-way analysis of variance as described in example 2.

### Vehicles used were:

Vehicle 1 (used in the groups 1 and 2): 30% hydroxypropyl-β-cyclodextrin and 0.3% NaCl (w/v), 5% ethanol (v/v) in Aqua sterilisata, pH 7.4.
Vehicle 2 (used when preparing dosing solutions of the groups 4, 5 and 6): 30% hydroxypropyl-β-cyclodextrin and 0.3% NaCl (w/v) in Aqua sterilisata, pH 7.4.
Vehicle 3 (used when preparing a dosing solution of the group 3): benzyl benzoate / ricinus oil (B/R) 1+4, v/v.

### Results

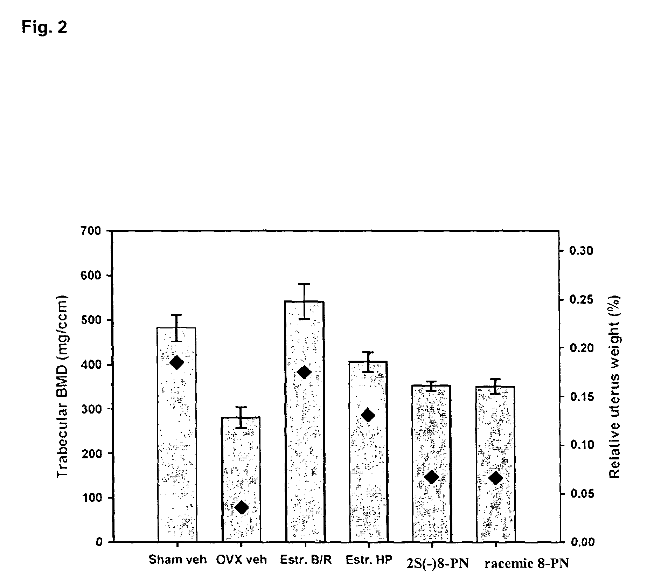

The results are shown in FIG 2 and in the following tables:

**Table 6**

| Body weight (g). | | | | |
|---|---|---|---|---|
| Group | | Body weight at The beginning (g) | Body weight at The end (g) | The change in Body weight (g) |
| | n | | | |
| | | (bw0) | (bw4b) | (bw4b-bw0) |
| Sham vehicle | 6 | 274(7) | 284(8) | 10(2) |
| OVX vehicle | 6 | 273(10) | 335(9) | 62(2) |
| Estradiol B/R | 6 | 274(7) | 291(9) | 17(4) |
| Estradiol HP | 6 | 275(6) | 285(9) | 10(4) |
| 2S(-)8PN | 8 | 264(5) | 289(6) | 24(2) |
| race.8PN | 8 | 262(7) | 289(8) | 27(2) |

**Table 7**

| Relative uterus weight | | | | | | | |
|---|---|---|---|---|---|---|---|
| Group | | Mean | SE | STD | Median | Min | MAX |
| | n | | | | | | |
| Sham vehicle | 6 | 0.185 | 0.021 | 0.052 | 0.179 | 0.125 | 0.254 |
| OVX vehicle | 6 | 0.036 | 0.001 | 0.004 | 0.037 | 0.031 | 0.040 |
| Estradiol B/R | 6 | 0.175 | 0.005 | 0.013 | 0.178 | 0.151 | 0.186 |
| Estradiol HP | 6 | 0.131 | 0.016 | 0.039 | 0.120 | 0.105 | 0.210 |
| 2S(-)8PN | 8 | 0.067 | 0.003 | 0.009 | 0.067 | 0.054 | 0.080 |
| race.8PN | 8 | 0.066 | 0.006 | 0.017 | 0.065 | 0.050 | 0.099 |

**Table 8**

| Trabecular density at left proximal tibia (mg/cm3). | | | | | | | |
|---|---|---|---|---|---|---|---|
| Group | | Mean | SE | STD | Median | Min | MAX |
| | n | | | | | | |
| Sham vehicle | 6 | 482 | 30 | 73 | 491 | 363 | 560 |
| OVX vehicle | 6 | 280 | 23 | 57 | 265 | 215 | 377 |
| Estradiol B/R | 6 | 542 | 39 | 95 | 577 | 364 | 624 |
| Estradiol HP | 6 | 406 | 22 | 53 | 383 | 367 | 501 |
| 2S(-)8PN | 8 | 352 | 11 | 31 | 352 | 309 | 393 |
| race.8PN | 8 | 351 | 16 | 46 | 351 | 289 | 415 |

Comparing the effects of 2S(-)8-Prenylnaringenin and racemic 8-Prenylnaringenin on inhibition of OVX-induced bone loss, the decrease of OVX-induced body weight gain, and the increase in uterine weight to estradiol given in the same vehicle (HP-β-CD), 2S(-)8-Prenylnaringenin and racemic 8-Prenylnaringenin had a much lower effect on the uterus and body weight than estradiol at the same bone effective dose. This observation is in agreement with the outcome of the previous study (see example 2).

The effects of racemic 8-Prenylnaringenin were similar to 2S(-)8-Prenylnaringenin.

### Description of the Figures

Fig.1 shows the trabecular bone mineral density (grey columns) and the relative uterine weight (black square) of rats which were either sham-operated and treated with vehicle (30% hydroxypropyl-β-cyclodextrin) or ovariectomized (ovx) and treated with the vehicle or 0.67 mg/kg, 1.77 mg/kg or 18 mg/kg 8-Prenylnaringenin.
Fig. 2 shows the trabecular bone mineral density (grey columns) and the relative uterine weight (black square) of rats treated as described in example 3. 8-PN designates 8-Prenylnaringenin and Estr. designates estradiol hemihydrate.
Fig. 3 shows the respective heights of luminal epithelium at nominal doses of 1, 3, and 30 mg/kg 2S(-)8PN.

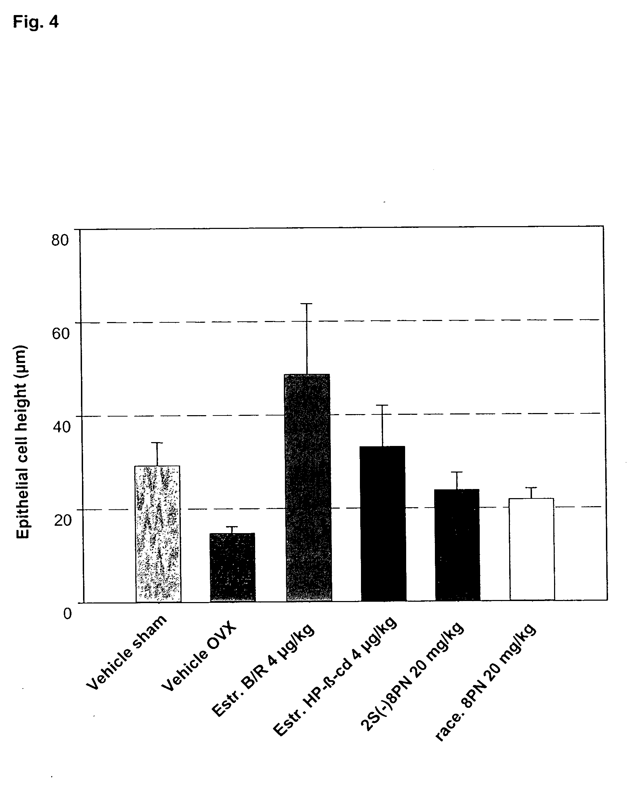

Fig. 4 shows the respective heights of luminal epithelium of rats treated as described in example 3.

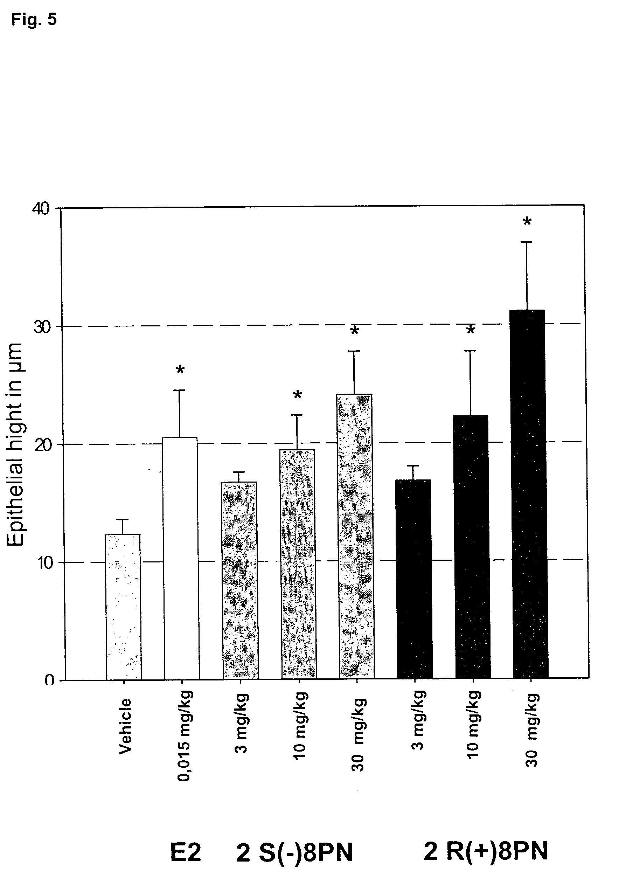

Fig. 5 shows a comparison of 2S(-)8Prenylnaringenin and 2R(+)8Prenylnaringenin. Shown are the heights of luminal epithelium of rats treated with 0,015 mg/kg estradiol hemihydrate, 3/10/30 mg/kg 2S(-)8PN or 3/10/30 mg/kg 2R(+)8PN.

## Claims

1. Method to synthesize 8-Prenylnaringenin comprising the steps:
a. diacetylation of naringenin
b. prenylation using tributylphosphine and diisopropylazodicarboxylate
c. rearrangement of prenyl side chain using Europium(III)-fod and
d. solvolysis using K₂CO₃ in methanol/H₂O
whereby said steps do not include a chromatography.

2. 8-Prenylnaringenin preparation produced by the method according to claim 1

3. Use of 8-Prenylnaringenin for the production of a medicament for the prevention and treatment of hormone dependent osteoporosis of peri- and postmenopausal symptoms in women wherein the 8-Prenylnaringenin preparation used for said production is at least 95% pure.

4. Use of 8-Prenylnaringenin according to claim 2 for the production of a medicament for the prevention and treatment of hormone dependent osteoporosis and of peri- and postmenopausal symptoms in women.

5. Use of 8-Prenylnaringenin for the production of a medicament for the prevention and treatment of hormone dependent osteoporosis of peri- and postmenopausal symptoms in women wherein the effect of said medicament on uterus proliferation is less than 1/5 of that of estradiol and the doses of 8-Prenylnaringenin and estradiol which are compared are equieffective on bone mass reduction
